# EUROPEAN PATENT APPLICATION

(11) **EP 2 163 330 A2**
(43) Date of publication of application: **17.03.2010**
(21) Application number: 09252071.7
(22) Date of filing: 26.08.2009
(51) Int. Cl.: B22F 3/11, B22F 9/24

(54) **Mixtures for forming porous constructs**

(30) Priority: 27.08.2008 US 92199 P
(71) Applicant: DePuy Products, Inc., Warsaw, IN 46581 (US)
(72) Inventor: Liu, Hengda D., Warsaw, IN 46580 (US); King, Richard S., Warsaw, IN 46580 (US)
(74) Representative: Tunstall, Christopher Stephen

(57) **Abstract**

Provided are methods comprising at least one metal powder with an extractable material and a composition comprising a polyol, a hydrophilic polymer, or both in order to form a mixture in which the metal powder and the extractable material assume respective positions. The composition functions as a homogenizing agent that allows the mixture to remain well-mixed for extended periods of time under ambient conditions. Also provided are green bodies and porous constructs, including implants, that are made in accordance with the disclosed methods. The green bodies and porous constructs have a substantially uniform porosity that is at least partially attributable to the ability of the composition to maintain the metal powder and the extractable material in their respective positions prior to sintering.

## Description

### CROSS REFERENCE TO RELATED APPLICATIONS

The present application claims priority to U.S. Provisional App. No. 61/092,199, filed August 27, 2008, the entire contents of which are hereby incorporated in their entirety.

### TECHNICAL FIELD

The present invention pertains to, among other things, the preparation of mixtures that can be used for the production of porous metal constructs.

### BACKGROUND

Porous metal constructs are widely used as, among other things, orthopedic implants, supports for catalysts, bone growth substrates, and filters. The "space holder" method is a well known process for making metallic foam structures and employs dissolvable or otherwise removable space-holding materials that are combined with metallic powders and subsequently removed from the combination by various methods, including heat evaporation or liquid dissolution, leaving behind a porous matrix formed from the metallic powder. The porous matrix material is then sintered to further strengthen the matrix structure. Numerous variations on the space holder concept are known in the art. *See, e.g.,* U.S. Pat. Nos. 3,852,045; 6,849,230; U.S. Pub. Nos. 2005/0249625; 2006/0002810.

Preferably, the mixture that results from the combination of space-holding materials and metallic powder features an even spatial distribution of the respective particle types. The positioning of the space-holding materials determines where, following processing to remove the space holder, pores will be present; therefore, a homogeneous distribution of space-holding particles will yield a construct having an even distribution of pores. Structural uniformity can provide numerous benefits. For example, orthopedic implants having uniform porosity are more likely to possess mechanical stability and to permit homogeneous ingrowth of bone and tissue, thereby leading to improved biological fixation.

Water or organic solvents may be used in small, controlled quantities to ensure a well-mixed combination of metallic powder and space holder material. However, water and organic solvents can gradually evaporate if the combination is stored for several days without commencing additional processing steps, and such evaporation can result in the segregation of the respective constituents of the combination. Other methods are said to involve the mixture of space holder material with liquid organics such as alcohols, isoparafinic solvents, acetone, or polyethylene glycol, followed by addition of metal powder in order to form mixtures that may be used for the preparation of porous metal articles. *See, e.g.,* U.S. Pub. Nos. 2006/0002810, 2006/0228247. However, organic solvents may be undesirably toxic, expensive, combustible, and/or volatile, while the high viscosity of polyethylene glycol may make mixing difficult.
There exist other drawbacks to such methods that justify a need for additional techniques for the preparation of mixtures that can be used to form constructs having uniform or otherwise controllable porosity.

### SUMMARY OF THE INVENTION

One aspect of the present invention provides methods comprising combining at least one metal powder with an extractable material and a composition comprising a polyol, a hydrophilic polymer, or both, thereby forming a mixture in which the metal powder and the extractable material assume respective positions. The present methods may further comprise shaping the mixture into a shaped object and compacting the shaped object to form a green body. The extractable material may be removed from the green body, and the green body may be sintered to form a porous metal construct for use as, for example, an implant. Also disclosed are green bodies and implants that are formed in accordance with such methods.

### BRIEF DESCRIPTION OF THE DRAWINGS

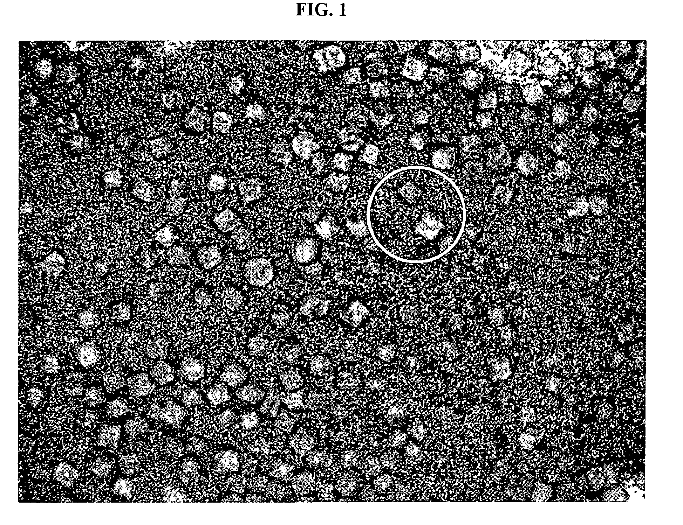

FIGS. 1-3 illustrate the tendency of a mixture comprising metal powder, space holder material, and reverse osmosis (RO) water to segregate after storage for four days following initial preparation of such mixture, as compared with the ability of mixtures that are prepared in accordance with the present invention to remain well-mixed up to and exceeding the same period of time.

### DETAILED DESCRIPTION OF ILLUSTRATIVE EMBODIMENTS

The present invention may be understood more readily by reference to the following detailed description taken in connection with the accompanying figures and examples, which form a part of this disclosure. It is to be understood that this invention is not limited to the specific products, methods, conditions or parameters described and/or shown herein, and that the terminology used herein is for the purpose of describing particular embodiments by way of example only and is not intended to be limiting of the claimed invention.

In the present disclosure the singular forms "a," "an," and "the" include the plural reference, and reference to a particular numerical value includes at least that particular value, unless the context clearly indicates otherwise. Thus, for example, a reference to "a polyol" is a reference to one or more of such polyols and equivalents thereof known to those skilled in the art, and so forth. When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive; as another example, the phrase "about 8%" preferably refers to a value of 7.2% to 8.8%, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", and the like. In addition, when a list of alternatives is positively provided, such listing can be interpreted to mean that any of the alternatives may be excluded, e.g., by a negative limitation in the claims. For example, when a range of "1 to 5" is recited, the recited range may be construed as including situations whereby any of 1, 2, 3, 4, or 5 are negatively excluded; thus, a recitation of "1 to 5" may be construed as "1 and 3-5, but not 2", or simply "wherein 2 is not included."

The disclosures of each patent, patent application, and publication cited or described in this document are hereby incorporated herein by reference, in their entirety.

The present invention pertains to, among other things, methods for forming mixtures that have and can retain a uniform distribution of metallic particles relative to particles of extractable material, as well as green bodies and sintered porous metal constructs (including implants) that are produced in accordance with such methods. In contrast with previously-existing processes, the present methods may employ safe, biocompatible, odorless, inexpensive, low volatility homogenizing agents and do not otherwise involve potentially harmful or inefficient processing steps. For example, the methods disclosed in U.S. Pub. Nos. 2006/0228247 involve the use of liquid organics such as alcohols, isoparafinic solvents, acetone, or polyethylene glycol as homogenizing agents; these agents are used to coat particles of space holder material, to which metal powder is subsequently added. The prescribed homogenizing agents are undesirably toxic, combustible, volatile, and/or high viscosity, which can present dangers or processing challenges during large-quantity product manufacture. Certain organic agents are also likely to evaporate relatively quickly from particle mixtures, thereby preventing long-term storage prior to subsequent processing steps. Furthermore, the step of addition of metal powder to the coated particles of space holder material can present safety issues, as the likelihood is high that metal particles will become airborne during the addition step. Also, the combination of metal powder and coated space holder material is said to be accomplished through the use of a v-blender, on which residual deposits are likely to form; the removal of such deposits may be difficult and will lower the efficiency of the process. As described herein, the present invention suffers from none of these drawbacks.

Provided are methods comprising combining at least one metal powder with an extractable material and a composition comprising a polyol, a hydrophilic polymer, or both, thereby forming a mixture in which the metal powder and the extractable material assume respective positions. The use of polyols and/or hydrophilic polymers to prepare the present mixtures confers numerous advantages. Such compounds are typically safe, biocompatible, odorless, inexpensive, and are characterized by low volatility. As demonstrated herein, the homogenizing agents of the present invention allow the metal powder and the extractable material to maintain their respective positions within the mixture for extended periods of time prior to any additional processing steps. Furthermore, unlike the prior art methods, the present methods need not (but nonetheless may, if desired) comprise the step of first combining a homogenizing agent with an extractable material, followed by adding a metal powder to the combination of the homogenizing agent and the extractable material, thereby reducing the probability that fine metal powder will become airborne and present a safety hazard during the manufacturing process. These and other advantages will become readily apparent in the present disclosure.

In one embodiment of the present methods, each of the metal powder, extractable material, and composition comprising a polyol, a hydrophilic polymer, or both may be mixed together in a single step. In another embodiment, a subcombination of the metal powder and extractable material may be formed prior to combining the metal powder and the extractable material with the composition. The subcombination may be formed as a substantially homogeneous blend of the metal powder and the extractable material. As used herein, a "substantially homogenous" mixture or blend of two particulate materials is characterized by a mostly even spatial distribution of the particles of the two materials relative to one another; when the subcombination comprises a major component and a minor component, the mixture may be a substantially uniform dispersion of the particles comprising the minor component among the particles comprising the major component. Those skilled in the art may readily appreciate various methods for forming a substantially homogenous mixture of particles, including one or more of blending, shaking, and stirring. Other techniques for forming a mixture of the metal powder and an extractable material will also be readily appreciated. *See, e.g.,* U.S. Pat. Nos. 3,852,045, 6,849,230; U.S. Pub. Nos. 2005/0249625, 2006/0002810. The mixture of the metal powder, extractable material, and composition comprising a polyol, a hydrophilic polymer, or both may also be formed using blending, shaking, stirring, or any other suitable technique. Preferably, formation of the mixture results in a substantially homogenous blend of the respective constituents. In one embodiment, preparation of the subcombination and/or the mixture of the metal powder, extractable material, and composition may be accomplished by agitating the ingredients together in a closed container using a conventional shaker. Unlike the prior art methods, which made use of a v-blender, the use of a shaker in accordance with the present invention is convenient and safe: a shaker is readily maintained and cleaned in between batches, and a closed container precludes the potentially dangerous dispersion of metal powder particles into the ambient atmosphere.

The subcombination may comprise metal powder in an amount that is about 5 percent by volume to about 45 percent by volume, preferably about 15 percent by volume to about 40 percent by volume, the balance of the subcombination comprising the extractable material. Once the extractable material is removed from the green body that is formed from the mixture of the metal powder, extractable material, and composition in later stages of the present methods, the resulting porosity of the green body may be about 55% to about 95%, preferably about 60% to about 85%. The removal of the extractable material is described more fully *infra.*

The composition with which the metal powder and extractable material are combined may comprise a polyol. The polyol may be present in the composition in an amount that constitutes about 5 percent by volume to about 80 percent by volume, about 10 percent by volume to about 70 percent by volume, about 20 percent by volume to about 60 percent by volume, or about 25 percent by volume to about 50 percent by volume. The balance of the composition may comprise water. When the composition comprises one or more polyols, the solution viscosity of the composition may be about 2 to about 20 centistokes at 20°C. Polyols are well known among those skilled in the art and constitute alcohols comprising two or more hydroxyl groups. In accordance with the present invention, polyols comprising three or more hydroxyl groups are preferred; for example, glycerol represents a highly preferred polyol for use in forming the present compositions. Other, nonlimiting examples of polyols include sorbitol, mannitol, xylitol, inositol, polyol sucrose, lactitol, maltitol, ethylene glycol, propane-1,2-diol, propane-1,3-diol, butane-1,4-diol, butane-1,3-diol, butane-2,3-diol, butene-1,4-diol, a pentanediol isomer, pentenediol, pentinediol, a hexanediol isomer, hexenediol, hexinediol, a heptanediol diol isomer, heptenediol, heptinediol, an octanediol isomer, octenediol, octinediol, trimethylol propane, pentaerythritol, or any combination thereof. Other suitable polyols may be readily identified by those skilled in the art.

The composition with which the metal powder and extractable material are combined may also or alternatively comprise a hydrophilic polymer. The hydrophilic polymer may be present in the composition in an amount that constitutes no more than about 5 percent by volume, no more than about 3 percent by volume, or no more than about 2 percent by volume of said composition. The balance of the composition may comprise water. When the composition comprises one or more hydrophilic polymers, the solution viscosity of may be about 5 to about 25 centistokes at 20°C. Hydrophilic polymers are well known among those skilled in the art. Nonlimiting examples of suitable hydrophilic polymers include polyvinyl alcohols, polyvinyl pyrrolidone, polyethylene oxide, sodium salts of polyacrylic acid, sodium salts of carboxyl methyl cellulose, sodium salts of alginic acid, sodium salts of hyaluronic acid, polyether polyol, polyether polyol modified with hydrophilic vinyl monomer, polytetramethylene polyol, or any combination thereof.

When the composition comprising a polyol, a hydrophilic polymer, or both comprises both one or more polyols and one or more hydrophilic polymers, the total complement of polyol and the total complement of hydrophilic polymer are preferably present in amounts that correspond to the respective ranges recited above. The balance of such compositions comprising both one or more polyols and one or more hydrophilic polymers may comprise water.

The amount of composition comprising a polyol, a hydrophilic polymer, or both in the mixture may be about 0.3% to about 1.5% by volume. In preferred embodiments, the amount of composition comprising a polyol, a hydrophilic polymer, or both in the mixture may be about 0.6% to about 0.75% by volume, the balance of the mixture comprising the metal powder and extractable material.

The metal powder may comprise any biocompatible metal, nonlimiting examples of which include titanium, a titanium alloy (e.g., Ti-6Al-4V), a cobalt-chromium alloy, aluminum, molybdenum, tantalum, magnesium, niobium, zirconium, stainless steel, nickel, tungsten, or any combination thereof. In accordance with known methods for forming porous constructs using metal powders, it will be readily appreciated that the metal powder particles may be substantially uniform or may constitute a variety of shapes and sizes, e.g., may vary in terms of their three-dimensional configuration and/or may vary in terms of their respective major dimension. Measured with respect to a given particle's major dimension, particle size may be from about 20 µm to about 100 µm, from about 25 µm to about 50 µm, and from about 50 µm to about 80 µm. The metal powder particles may be spheroids, roughly cylindrical, platonic solids, polyhedrons, plate- or tile-shaped, irregularly shaped, or any combination thereof. In preferred embodiments, the metal powder comprises particles that are substantially similarly shaped and substantially similarly sized.

The extractable material may be a material that is soluble in an aqueous solvent, an organic solvent, or both, and may include a salt, a sugar, a solid hydrocarbon, a urea derivative, a polymer, or any combination thereof. Nonlimiting examples include ammonium bicarbonate, urea, biuret, melamine, ammonium carbonate, naphthalene, sodium bicarbonate, sodium chloride, ammonium chloride, calcium chloride, magnesium chloride, aluminum chloride, potassium chloride, nickel chloride, zinc chloride, ammonium bicarbonate, sodium hydrogen phosphate, sodium dihydrogen phosphate, potassium dihydrogen phosphate, potassium hydrogen phosphate, potassium hydrogen phosphite, potassium phosphate, magnesium sulfate, potassium sulfate, alkaline earth metal halides, crystalline carbohydrates (including sucrose and lactose or other materials classified as monosaccharides, disaccharides, or trisaccharides), polyvinyl alcohol, polyethylene oxide, a polypropylene wax (such those available from Micro Powders, Inc., Tarrytown, NY, under the PROPYLTEX® trademark), sodium carboxymethyl cellulose (SCMC), or any combination thereof.

The particles constituting the extractable material may be substantially uniform or may constitute a variety of shapes and sizes, e.g., may vary in terms of their three-dimensional configuration and/or may vary in terms of their respective major dimension.

The extractable material can be present in a wide variety of particle sizes and particle size distributions suitable to produce a desired pore size and pore size distribution. Certain preferred particle size ranges are from about 200 µm to about 600 µm, from about 200 µm to about 300 µm, and from about 425 µm to about 600 µm. The extractable material particles may be spheroids, roughly cylindrical, platonic solids, polyhedrons, plate- or tile-shaped, irregularly shaped, or any combination thereof. In preferred embodiments, the extractable material comprises particles that are substantially similarly shaped and substantially similarly sized. Because the size and shape of the pores of the porous body that is produced from the mixture of the metal powder, the extractable material, and the composition roughly correspond to the size and shape of the particles of the extractable material, one skilled in the art will readily appreciate that the characteristics of the particles of the extractable material may be selected according to the desired configuration of the pores of the resulting porous product.

In accordance with the present invention, when the extractable material comprises particles that are substantially similarly shaped and substantially similarly sized, the porosity of the porous body that is formed using the extractable material of this type will be substantially uniform. As demonstrated in Examples 1 and 2, *infra,* the use of an inventive composition comprising a polyol, a hydrophilic polymer, or both permits the formation of a stable mixture of the composition, the metal powder, and the extractable material and more homogeneity of structure. Prior techniques did not produce porous constructs having the degree of uniformity that can be achieved in accordance with the present methods, even when the extractable materials were selected for the uniformity of particle size and shape. Thus, not only does the present invention provide for stable mixtures of metal powder and extractable materials that do not segregate during prolonged periods of storage (for example, for at least four days, at least one week, at least ten days, or at least two weeks) following the combination of the metal powder and extractable material with the composition comprising a polyol, a hydrophilic polymer, or both, but the present invention also provides porous bodies (including both green bodies and sintered bodies) that possess substantially uniform porosity when such porous bodies are produced in accordance with the disclosed methods. "Substantially uniform porosity" refers to the characteristic of a green body, or a porous body that is produced from a green body, whereby the porosity does not significantly vary across the body. For example, in some embodiments the porosity of the body varies by a standard deviation of no more than about 3, where porosity is measured in terms of percentage. In yet other embodiments, the porosity of the body varies by a standard deviation of no more than about 2.

The present methods may further comprise shaping the mixture of the at least one metal powder, the extractable material, and the composition comprising a polyol, a hydrophilic polymer, or both, in order to form a shaped object, and compacting the shaped object to form a green body. The present invention is also directed to green bodies that are produced in accordance with any process described herein. The shaping process can comprise filling a mold with the mixture, the mold having at least roughly the three-dimensional parameters of the desired final porous product, allowing for subsequent processing steps such as machining. In other embodiments, the mold need not be designed to produce near-net shape parts or parts whose molded form resembles the desired final, sintered part; molds may produce generic shapes, such as bars, rods, plates, or blocks, that may be subsequently machined in the green state to produce a part that after sintering-induced shrinkage closely approximates the desired shape of the final product, with optional machining of the sintered part. Molds and mold assemblies for such purposes are well known among those skilled the art and may allow for the preparation of bodies that are, for example, spheroid, ovoid, hemispherical, cuboid, cylindrical, toroid, conical, concave hemispherical (i.e., cup-shaped), plate- or tile-shaped, irregular, or that adopt any other desired three-dimensional conformation. Once formed from the mixture in accordance with any aspect(s) of the preceding description, the resulting shaped object may be compacted to form the green body. The shaped object is compacted while contained within a mold assembly. Compacting may be uniaxial, multi-axial, or isostatic. In preferred embodiments, a cold isostatic press is used to compact the shaped object into the green body. Following the compacting procedure, the resulting green body may be removed from the mold and processed. Processing may include machining or otherwise refining the shape of the green body.

Whether or not machining is performed after compacting, the green body may then be exposed to a solvent in which the extractable material is soluble. As indicated above, the extractable material may be soluble in an aqueous solvent, an organic solvent, or both. The exposure of the green body to the solvent may comprise immersing the green body in the solvent, for example, by immersing the green body in a bath comprising the solvent for a time sufficient to remove at least some of the extractable material. Depending on various factors such as the type of solvent chosen relative to the identity of the extractable material, the temperature of the solvent, and the time of exposure to the solvent, the removal of extractable material from the green body can range from partial to complete. The exposure of the green body to the solvent in which the extractable material is soluble preferably removes the extractable material from at least the surface of the green body to a depth at least about 1 mm, at least about 3 mm, at least about 5 mm, at least about 7 mm, or at least about 10 mm from any given surface of the green body.

The composition comprising the polyol, hydrophilic polymer, or both may also be soluble in an aqueous solvent, an organic solvent, or both. When such is the case, the composition may be removed from the green body during the process of exposing the green body to a solvent in which the extractable material is soluble. For example, if the composition comprises glycerol, the composition can be removed from the green body using water, and if the extractable material is soluble in an aqueous solvent, then the extractable material and the composition may be removed from the green body during a single processing step. The solubility of the composition may permit the effectively complete removal thereof from the green body, and under such circumstances no contamination of the green body (or any sintered porous body produced from the green body) by the composition will occur.

In another embodiment, the extractable material is insoluble in aqueous or organic solvent, and is removable under heating conditions. In such circumstances, after the formation of the green body via compacting (and whether or not machining is performed following compacting), the green body may be heated for a time and under conditions effective to evaporate at least some of the extractable material yet substantially maintain the metal powder in its position in the green body. Depending on various factors such as the identity of the extractable material, the temperature of the heating environment, and the time of heating, the removal of extractable material from the green body can range from partial to complete, and the heating of the green body preferably removes the extractable material from at least the surface of the green body, down to at least about 5% of the total depth of the green body. Preferably, the thermal removal of extractable material is performed at temperatures much lower than sintering temperature, in order to avoid contamination of the green body material with C, N, O, or H from organic space holders. For example, the thermal removal of extractable material may occur at less than about 100°C, which is sufficient to cause the decomposition of some extractable materials, such as ammonium bicarbonate.

The removal of the extractable material under heating conditions may occur before, after, or contemporaneously with the removal of the composition. The composition may be removable under the same heating conditions as are effective for the removal of the extractable material. In other instances, the composition is soluble in an aqueous solvent, organic solvent, or both, and the removal of the composition, for example, by immersion of the green body in a bath comprising the appropriate solvent or solvents, may be performed prior to or following the removal of the extractable material by heating.

As indicated above, once the extractable material is removed from the green body, the resulting porosity of the green body may be about 50% to about 95%, preferably about 60% to about 85%.

Following the removal of the composition and the extractable material from the green body, the present methods may further comprise sintering the green body. Sintering is typically performed in a vacuum furnace and those skilled in the art will readily appreciate the appropriate conditions for sintering a green body comprising a metal powder. Sintering may be followed by additional processing steps, including machining to refine the shape characteristics of the sintered body. The present invention is also directed to sintered porous bodies that are made in accordance with the described methods. The sintered porous bodies may comprise implants, such as orthopedic implants used to replace or repair any damaged or otherwise compromised element or portion of an element of a skeletal system. The present implants are characterized by substantially uniform porosity and therefore provide numerous benefits associated with this property, such as mechanical stability and homogeneous ingrowth of bone and tissue, thereby leading to improved biological fixation.

### EXAMPLE 1 - Stability of Inventive Mixtures

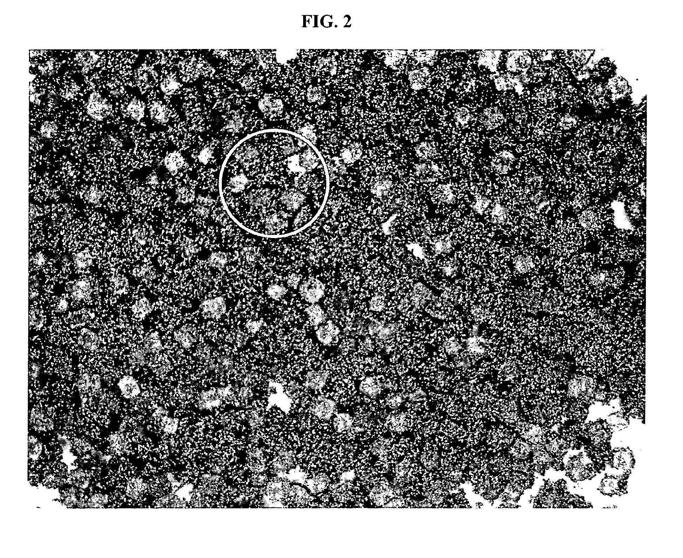

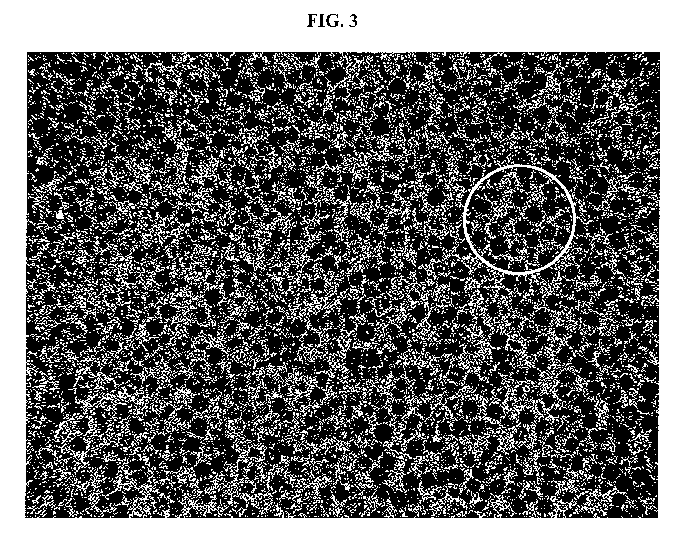

A first mixture comprising titanium metal powder, NaCl extractable material, and glycerol solution was prepared. A second mixture comprising titanium metal powder, NaCl and reverse osmosis (RO) water was also prepared, and both mixtures were placed under a fume hood in separate open containers. Both mixtures were left undisturbed under the fume hood for a period of four days. As shown in FIG. 1, the mixture that was prepared using RO water did not remain well-mixed following the four-day period; the circled portion of FIG. 1 clearly shows the segregation of NaCl particles from the particles of titanium. In contrast, the mixture that was prepared using glycerol solution remained well-mixed following the same four-day period, with no visible segregation of the salt particles from the metal powder (circled portion of FIG. 2). A third mixture, comprising titanium metal powder, NaCl extractable material, and glycerol solution, was prepared and placed under a fume hood in an open container and left undisturbed for a period of one week. The mixture was subsequently compacted using a cold isostatic press before the image provided in FIG. 3 was taken. As may be easily observed from FIG. 3 (circled portion), the salt portion of the mixture remained mixed with Ti powder; as will be readily appreciated by one of ordinary in the art, a homogeneous, compacted mixture can only result if the uncompacted mixture was also homogenous.

Therefore, mixtures of metal powder, extractable material, and a composition comprising a polyol, a hydrophilic polymer, or both remain well-mixed following the combination of the respective ingredients, even when stored in a container that is exposed to the ambient atmosphere for at least as long as seven days. In contrast, mixtures that are prepared using traditional methods, such as combining the metal powder and extractable material with water, do not remain as well-mixed and evince segregation between the metal particles and the particles of extractable materials, respectively.

### EXAMPLE 2 - Porosity of Sintered Bodies

Three separate mixtures were prepared. The first mixture comprised a combination of titanium powder, NaCl, and 25% by volume glycerol solution as a homogenizing agent. The second mixture included the same ingredients but used a 50% glycerol solution instead of a 25% solution, and the third mixture also included titanium powder and NaCl, but made use of reverse osmosis (RO) water as the homogenizing agent rather than glycerol. Each of the three mixtures were subsequently compacted, exposed to aqueous solvent to remove the NaCl particles, and sintered to form a porous body. The three samples were otherwise alike with respect to titanium powder particle size, salt particle size, green porosity, the total quantity of homogenizing agent combined with the titanium powder and NaCl, compaction pressure, sintering temperature, and sintering time.

The porosity of each of the three samples was measured by an imaging process using Image-Pro®Plus software (Media Cybernetics, Inc., Silver Spring, MD). Each sample was sectioned into four pieces and each piece was cold mounted and then grinded and polished to reveal the original porous structure. Two optical microscope images (50x magnification) were taken from each section, providing a total of eight images from each sample. In the acquired images, metal scaffold material appears dark and pores appear white, which permits measurement of the metal foam porosity based on the areas respectively occupied by the metal and pores. As shown in Table 1, below, the samples prepared using glycerol solution each feature porosities that have a standard deviation that is only about one half of the standard deviation of the porosity of the sample that is prepared using RO water.

**TABLE 1**

| **Porosity (%)** | **Homogenizing Agent** | | |
|---|---|---|---|
| | **25% Glycerol** | **50% Glycerol** | **RO Water** |
| Maximum | 73.63 | 74.38 | 74.94 |
| Minimum | 68.75 | 68.51 | 66.39 |
| Range | 4.88 | 5.87 | 8.55 |
| Mean | 71.69 | 72.08 | 71.64 |
| Standard Deviation | ±1.80 | ±1.79 | ±3.48 |

The sample that was prepared using 25% glycerol solution possessed the narrowest range for measured porosity, indicating that among the tested samples, the highest degree of uniformity was achieved when 25% glycerol solution was combined with titanium powder and NaCl particles in forming the porous body.

## Claims

1. A method comprising combining at least one metal powder with an extractable material and a composition comprising a polyol, a hydrophilic polymer, or both, thereby forming a mixture in which said metal powder and said extractable material assume respective positions.

2. The method according to claim 1 wherein a subcombination of said metal powder and said extractable material is formed prior to combining said metal powder and said extractable material with said composition.

3. The method according to claim 2 wherein said subcombination comprises metal powder in an amount that is about 5 percent by volume to about 45 percent by volume of said subcombination.

4. The method according to claim 2 wherein said subcombination is formed as a substantially homogeneous blend of said metal powder and said extractable material.

5. The method according to claim 1 further comprising subjecting said mixture to one or more of blending, shaking, and stirring.

6. The method according to claim 1 wherein said composition comprises a polyol in an amount that constitutes about 10 percent by volume to about 70 percent by volume of said composition.

7. The method according to claim 1 wherein said polyol is glycerol, sorbitol, mannitol, xylitol, inositol, polyol sucrose, lactitol, maltitol, ethylene glycol, propane-1,2-diol, propane-1,3-diol, butane-1,4-diol, butane-1,3-diol, butane-2,3-diol, butene-1,4-diol, butine-1,4-diol, pentane-1,5-diol, an isomeric pentanediol, pentenediol, or pentinediol, hexane-1,6-diol or an isomeric hexanediol, hexenediol or hexinediol, heptane-1,7-diol, an isomeric heptanediol, heptenediol or heptinediol, octane-1,8-diol, an isomeric octanediol, octenediol, or octinediol, trimethylol propane, pentaerythritol, or any combination thereof.

8. The method according to claim 1 wherein said polyol is glycerol.

9. The method according to claim 1 wherein said composition comprises a hydrophilic polymer in an amount that constitutes no more than about 5 percent by volume of said composition.

10. The method according to claim 1 wherein said hydrophilic polymer is a polyvinyl alcohol, polyvinyl pyrrolidone, polyethylene oxide, a sodium salt of polyacrylic acid, a sodium salt of carboxyl methyl cellulose, a sodium salt of alginic acid, a sodium salt of hyaluronic acid, polyether polyol, polyether polyol modified with hydrophilic vinyl monomer, polytetramethylene polyol, or any combination thereof.

11. The method according to claim 1 wherein said metal powder comprises titanium, a titanium alloy, a cobalt-chromium alloy, aluminum, molybdenum, tantalum, magnesium, niobium, zirconium, stainless steel, nickel, tungsten, or any combination thereof.

12. The method according to claim 1 wherein said extractable material comprises ammonium bicarbonate, urea, biuret, melamine, ammonium carbonate, naphthalene, sodium bicarbonate, sodium chloride, ammonium chloride, calcium chloride, magnesium chloride, aluminum chloride, potassium chloride, nickel chloride, zinc chloride, ammonium bicarbonate, sodium hydrogen phosphate, sodium dihydrogen phosphate, potassium dihydrogen phosphate, potassium hydrogen phosphate, potassium hydrogen phosphite, potassium phosphate, magnesium sulfate, potassium sulfate, alkaline earth metal halides, crystalline carbohydrates, polyvinyl alcohol, polyethylene oxide, polypropylene wax, sodium carboxymethyl cellulose, or any combination thereof.

13. The method according to claim 1 further comprising:
shaping said mixture into a shaped object; and,
compacting said shaped object to form a green body.

14. A green body prepared in accordance with the method of claim 13.

15. The method according to claim 13 further comprising processing said green body.

16. The method according to claim 13 further comprising heating said green body for a time and under conditions effective to evaporate at least some of said extractable material yet substantially maintain said metal powder in its position in said green body.

17. The method according to claim 13 further comprising exposing said green body to a solvent in which said extractable material is soluble.

18. The method according to claim 17 wherein said extractable material is soluble in an aqueous solvent, an organic solvent, or both.

19. The method according to claim 17 wherein said green body is immersed in said solvent.

20. The method according to claim 17 wherein said composition is soluble in said solvent.

21. The method according to claim 17 wherein the green body has a total porosity of about 50% to about 95%.

22. The method according to claim 21 wherein the green body has a substantially uniform porosity.

23. The method according to claim 22 wherein the porosity of said green body varies by a standard deviation of less than about 3.

24. The method according to claim 22 wherein the porosity of said green body varies by a standard deviation of less than about 2.

25. The method according to claim 16 further comprising sintering said green body.

26. An implant made in accordance with the method of claim 25.

27. A mixture made in accordance with the method of claim 1.
